# EUROPEAN PATENT APPLICATION

(11) **EP 3 653 646 A1**
(43) Date of publication of application: **20.05.2020**
(21) Application number: 18382808.6
(22) Date of filing: 15.11.2018
(51) Int. Cl.: C07K 16/28

(54) **METHOD AND KIT FOR THE DIAGNOSIS AND/OR PROGNOSIS OF INFLAMMATORY CONDITIONS**

(71) Applicant: Fundació Institut d'Investigació en Ciències de la Salut Germans Trias i Pujol, 08916 Badalona, Barcelona (ES)
(72) Inventor: SARRIAS FORNÉS, Maria Rosa, 08009 Barcelona (ES); ARAN CANALS, Gemma, 08291 Ripollet (ES); TÉLLEZ CABALLERO, Èrica, 08917 Badalona (ES); VILAPLANA MASSAGUER, Cristina, 08006 Barcelona (ES); BORRÁS SERRES, Francesc E., 08012 Barcelona (ES)
(74) Representative: Elzaburu S.L.P.

(57) **Abstract**

The present invention relates to a diagnostic and/or prognostic method of conditions that arise in an inflammatory context based on analysing the levels of CD5L in a sample using the monoclonal antibody produced by the hybridoma deposited at the BCCM and assigned Accession No. LMBP 12584CB and the monoclonal antibody produced by the hybridoma deposited at the BCCM and assigned Accession No. LMBP 12583CB. Also, the present invention relates to the monoclonal antibodies, to a kit comprising them and their use for the diagnosis and/or prognosis of conditions that arise in an inflammatory context.

SEQUENCE LISTING

<110>  FUNDACIÓ INSTITUT D'INVESTIGACIÓ EN CIÈNCIES DE LA SALUT
       GERMANS TRIAS I PUJOL

<120>  METHOD AND KIT FOR THE DIAGNOSIS AND/OR PROGNOSIS OF INFLAMMATORY
       CONDITIONS

<130>  EP-1969

<160>  1

<170>  PatentIn version 3.5

<210>  1
<211>  987
<212>  DNA
<213>  Homo sapiens

<400>  1
tctccatctg gagtgcggct ggtggggggc ctccaccgct gtgaagggcg ggtggaggtg      60

gaacagaaag gccagtgggg caccgtgtgt gatgacggct gggacattaa ggacgtggct     120

gtgttgtgcc gggagctggg ctgtggagct gccagcggaa cccctagtgg tattttgtat     180

gagccaccag cagaaaaaga gcaaaaggtc ctcatccaat cagtcagttg cacaggaaca     240

gaagatacat tggctcagtg tgagcaagaa gaagtttatg attgttcaca tgatgaagat     300

gctggggcat cgtgtgagaa cccagagagc tctttctccc cagtcccaga gggtgtcagg     360

ctggctgacg gccctgggca ttgcaaggga cgcgtggaag tgaagcacca gaaccagtgg     420

tataccgtgt gccagacagg ctggagcctc cgggccgcaa aggtggtgtg ccggcagctg     480

ggatgtggga gggctgtact gactcaaaaa cgctgcaaca agcatgccta tggccgaaaa     540

cccatctggc tgagccagat gtcatgctca ggacgagaag caacccttca ggattgccct     600

tctgggcctt gggggaagaa cacctgcaac catgatgaag acacgtgggt cgaatgtgaa     660

gatccctttg acttgagact agtaggagga gacaacctct gctctgggcg actggaggtg     720

ctgcacaagg gcgtatgggg ctctgtctgt gatgacaact ggggagaaaa ggaggaccag     780

gtggtatgca agcaactggg ctgtgggaag tccctctctc cctccttcag agaccggaaa     840

tgctatggcc ctggggttgg ccgcatctgg ctggataatg ttcgttgctc aggggaggag     900

cagtccctgg agcagtgcca gcacagattt tgggggtttc acgactgcac ccaccaggaa     960

gatgtggctg tcatctgctc aggatag                                         987

## Description

### FIELD OF THE INVENTION

The present invention relates to a diagnostic and/or prognostic method of conditions that arise in an inflammatory context. The present invention also relates to monoclonal antibodies and a kit comprising them, and their use for the diagnosis and/or prognosis of conditions that arise in an inflammatory context.

### BACKGROUND OF THE INVENTION

CD5L, a soluble protein belonging to the scavenger receptor cysteine-rich superfamily, is expressed mostly by macrophages in both lymphoid and inflamed tissues. The expression of this protein is transcriptionally controlled by liver X receptors, members of the nuclear receptor family that play major roles in lipid homeostasis. Research undertaken over the last decade has uncovered critical roles of CD5L as a pattern recognition receptor of bacterial and fungal components and in the control of key mechanisms in inflammatory responses, with involvement in processes such as infection, atherosclerosis, and cancer. The current knowledge of CD5L and its roles at the intersection between lipid homeostasis and immune response have involved considering its potential use as a diagnostic biomarker in a variety of diseases of inflammatory origin (Sanjurjo L, et al. 2015).

In blood, CD5L circulates in high concentrations (∼10 µg/mL) in association with IgM, and the plasma levels of both proteins are positively correlated. The IgM-CD5L interaction was initially discovered upon CD5L detection in IgM but not in IgG or IgA fractions of human serum (Tissot JD, et al. 1994).

Biomarkers have gained significant clinical value in medical practice and have recently been introduced into clinical patient management for the diagnosis, treatment stratification, and prognosis of diverse pathologies. As mentioned, hCD5L is detected in serum in relatively high amounts (µg/mL range), and a large-scale analysis in a healthy population revealed higher levels in women than in men (mean 6.06±2.1 µg/ml in women, 4.99±1.8 µg/ml in men) (Yamazaki T, et al. 2014). Interestingly, hCD5L peaked in women in their 20s and decreased with age (mean 6.75 ± 2.05 µg/ml at 20 years old, 4.91±1.57 at 70 years old (Yamazaki T, et al. 2014). Moreover, plasma levels of CD5L are altered in several conditions that arise in an inflammatory context. In this regard, mCD5L plasma levels increase up to 10-fold in mice infected with *M. tuberculosis* three weeks after infection, coinciding with peak colony forming units (CFU) numbers in spleen and lung (Sanjurjo L, et al. 2013). Likewise, septic shock induction with LPS and zymosan modulates mCD5L plasma levels in mice, resulting in increased levels 24 hours post injection.

Several proteomic studies using human plasma/serum, and synovial and bronchoalveolar lavage fluid have highlighted hCD5L protein as a putative biomarker for a number of inflammatory conditions (Table 1). The list of conditions is steadily increasing, ranging from atopic dermatitis (Kim WK, Et al. 2008) to Kawasaki disease (Yu HR, et al. 2009) and osteoarthritis (Balakrishnan L, et al. 2014). Interestingly, most of the studies focused on the value of hCD5L as a biomarker of liver disease (Armengol C, et al. 2013).

**Table 1. Proteomic studies that identify CD5L as a putative biomarker of diseases ^{(a)}.**

| **Disease** | **Origin of samples** | **CD5L levels**^{(b)} | **CD5L presumed function** | **Reference** |
|---|---|---|---|---|
| Asthma | BALF | ↑ | None | Wu, J. et al. 2005 |
| Liver cirrhosis in HCV infection | Serum | ↑ | Immune response to HCV infection | Gangadharan B. et al. 2007 |
| Liver cirrhosis and HCC in fatty liver disease | Serum | ↑ | Anti-apoptotic role, supporting hepatocyte regeneration | Gray, J. et al. 2009 |
| HCC in HCV infection | Serum | ↑ | Immune response to HCV infection | Sarvari, J. et al. 2013 |
| Chronic liver disease due to HCV infection | Serum | ↑ | None | Mera, K. et al 2014 |
| HCC vs cirrhosis | Serum | | None | Yamazaki, T. et al. 2014 |
| Atopic dermatitis | Plasma | ↑ | Antiapoptotic role. Promoting eosinophilia | Kim, WK. et al. 2008 |
| HCC development in NASH patients | Serum | ↑ | | Koyama 2017 |
| Kawasaki disease | Serum | ↑ | Antiapoptotic role. Dysregulation of apoptosis in coronary artery lesions | Yu, HR. et al. 2009 |
| Turner syndrome | Plasma | ↑ | None | Kolialexi A. et al. 2010 |
| CLI in diabetic patients | Plasma | ↑ | None | Hung, P-H. et al. 2011 |
| Pulmonary TB | Serum | ↑ | Macrophage recognition of Mtb | Xu, DD, et al. 2014 |
| Osteoarthritis | Synovial fluid | ↑ | None | Balakrishnan L. et al. 2014 |
| Extreme physical stress | Plasma | ↓ | Antiapoptotic role. Preventing stress-induced apoptosis. | Balfoussia E. et al. 2014 |
| Age-related macular degeneration | Serum | ↑ | None | lannacone 2016 |
| Crohn's disease | Serum | ↑ | | Ono 2017 |
| Psoriatic Arthritis | Serum | ↑ | | Cretu 2017 |
| Early onset preeclampsia | Plasma | ↑ | | Kolialexi 2017 |
| Amyotrophic lateral sclerosis with cognitive impairment | Plasma | ↓ | | Xu 2018 |
| Prostate Cancer | Serum | ↑ | | Totten 2018 |
| Systemic lupus erythematosus | Serum | ↑ | | Lai 2018 |

| | | | | |
|---|---|---|---|---|
| ^{(a)} Abbreviations: AD, atopic dermatitis; BALF, bronchoalveolar lavage fluid; CLI, critical limb ischemia; ELISA, enzyme-linked immunosorbent assay; HCC, hepatocarcinoma; HCV, hepatitis C virus; iTRAC, isobaric tag for relative and absolute quantitation; KD, Kawasaki disease; MS, mass spectrometry; MTB, Mycobacterium tuberculosis; NAFLD, non-alcoholic fatty liver disease; TB, tuberculosis; TS, Turner syndrome.. ^{(b)} ↓ decreased; ↑ increased. | | | | |

In the liver, chronic inflammation causes high morbidity and mortality worldwide. Hepatitis virus infection, alcohol abuse, and non-alcoholic fatty liver (NAFLD) are the main aetiologies associated with this disease. In this context, continuous inflammation as a result of liver damage leads to hepatic fibrosis, which frequently brings about cirrhosis and ultimately HCC.

The determination of a plasma biomarker of liver fibrosis or HCC would be of great relevance for the clinical management of these patients. In this context, proteomic analysis based on 2D gel electrophoresis identified enhanced levels of hCD5L in the sera of individuals with liver cirrhosis related to hepatitis C virus (HCV) infection as compared to healthy control serum, and this protein was proposed as a potential biomarker for assessing liver fibrosis (Gangadharan, B., et al. 2007).

A later study confirmed that serum levels of hCD5L are indicators of advanced liver fibrosis in HCV (Mera, K. et al. 2014). Also, on the basis of its proposed role in immune system regulation, hCD5L was thought to be most likely associated with viral infection rather than cirrhosis (Gangadharan, B., et al. 2007). In the context of HCV infection, a study on 19 HCV-positive patients that included 7 with cirrhosis and 5 with HCC suggested that CD5L might be a useful biomarker for early diagnosis of HCC in HCV cirrhotic patients (Sarvari, J., et al. 2013).

A more recent report showed that certain combinations of hCD5L indexes normalized to liver marker score distinguished HCC patients from non-HCC patients (mostly HCV) and thus could be applicable for HCC diagnosis (Yamazaki, T., et al. 2014). However, a proteomic approach applied in different stages of NAFLD identified and further validated hCD5L as an upregulated serum biomarker for cirrhotic NAFLD, but discarded hCD5L as a surveillance tool for HCC in these patients (Gray, J., et al. 2009).

These studies suggest that blood levels of hCD5L may serve as a biomarker of liver cirrhosis in HCV and NAFLD, and HCC only in HCV. Future studies are required to confirm the potential of hCD5L as a biomarker of liver damage/HCC and of other inflammatory diseases. Moreover, significant alterations of hCD5L levels point to a functional implication of this protein in these pathologies-a question that deserves further investigation.

Given the relevance of CD5L as biomarker, there is a need for reliable and easy to use diagnostic/prognostic tools. Currently, there is no *in vitro* Diagnostic Device (IVD) in the market to evaluate CD5L levels.

### DESCRIPTION OF THE INVENTION

The inventors have developed two IVDs intended for use in patient management. These IVDs are an ELISA kit to accurately measure the levels of CD5L in different samples; and a rapid diagnostic, qualitative kit (lateral flow immunochromatographic assay kit, IC kit), to measure the levels of CD5L in plasma or urine samples at the point of care.

The term "CD5L" as used herein refers to the soluble protein named CD5 antigen-like, Apoptosis inhibitor 6 (Api-6), Soluble protein alpha (Spalpha), Apoptosis inhibitor expressed by macrophages (AIM), CT-2, or IgM-associated peptide, which belongs to the scavenger receptor cysteine-rich (SRCR) superfamily. The protein sequence from several species is available in several protein databases, such as in Uniprot O43866_HUMAN *Homo sapiens;* Q9QWK4_MOUSE *Mus musculus;* A6QNW7_BOVIN *Bos taurus;* F7FUB7_MACMU *Macaca mulatta;* Q4KM75_RAT *Rattus norvegicus;* H2Q0B2_PANTR *Pan troglodytes;* F7HDX2_CALJA *Callithrix jacchus;* F1PAX5_CANLF *Canis lupus familiaris;* H0UZM6_CAVPO *Cavia porcellus;* G3R058_GORGO *Gorilla gorilla gorilla;* F1RN76_PIG *Sus scrofa;* A0A1E1GEV5_FELCA *Felis catus;* F7BXD8_HORSE *Equus caballus.*

Therefore, a first aspect of the present invention relates to a diagnostic and/or prognostic method comprising:
a. analysing the levels of CD5L in a sample using the monoclonal antibody produced by the hybridoma deposited at the BCCM and assigned Accession No. LMBP 12584CB and the monoclonal antibody produced by the hybridoma deposited at the BCCM and assigned Accession No. LMBP 12583CB;
b. comparing the levels of CD5L obtained in step (a) with those analysed in control samples; and
c. arriving to a diagnosis/prognosis where the levels in the sample are consistent with those in subjects who have been diagnosed previously.

BCCM is the Belgian Co-ordinated Collections of Micro-organisms in Universiteit Gent, Vakgroep Biomedische Moleculaire Biologie, Technologiepark, 927, 9052 Zwijnaarde, Belgium. Both hybridomas accessed the BCCM on 22 August 2018.

Preferably, the diagnostic and/or prognostic method of the present invention allows for the diagnostic and/or prognostic of inflammatory conditions or conditions that arise in an inflammatory context.

An aspect of the present invention is also a diagnostic and/or prognostic method comprising analysing the levels of CD5L in a sample using the monoclonal antibody produced by the hybridoma deposited at the BCCM and assigned Accession No. LMBP 12584CB.

An aspect of the present invention is also a diagnostic and/or prognostic method comprising analysing the levels of CD5L in a sample using the monoclonal antibody produced by the hybridoma deposited at the BCCM and assigned Accession No. LMBP 12583CB.

In contrast with prior methods and kits comprising antibodies for the diagnosis and/or prognosis of conditions that arise in an inflammatory context, this invention reaches 100% sensitivity and 80% specificity, which is a significantly higher value than the performances of methods and kits of the prior art.

The inventors have developed novel CD5L monoclonal antibodies. Said antibodies have the following advantages over prior art polyclonal antibodies, particularly for their use in prognostic/diagnostic methods and devices:
(a) polyclonal antibodies are not as good as monoclonal antibodies for quantification experiments since more than one antibody binds the same molecule and the signal is amplified in a non-reproducible way from batch to batch and from trial to trial;
(b) monoclonal antibodies consist of only one antibody subtype, which allows for the selection of the correct secondary antibody for detection, while this is not possible for polyclonal antibodies;
(c) hybridomas are a constant and renewable source once created, and all batches will be identical, increasing consistency and standardization of experimental procedures and results;
(d) monoclonal antibodies give less background. Specifically, detecting one target epitope means they are less likely to cross-react with other proteins; and
(e) homogeneity of monoclonal antibodies is very high relative to polyclonals. If experimental conditions are kept constant, results from monoclonal antibodies will be highly reproducible between experiments.

In a preferred embodiment of the diagnostic and/or prognostic method of the first aspect, the monoclonal antibody produced by the hybridoma deposited at the BCCM and assigned Accession No. LMBP 12583CB is labelled with biotin, conjugated to colloidal gold, colloidal silver, carbon, a visible or fluorescent dye, a magnetic particle, an enzyme such as Horseradish peroxidase, alkaline phosphatase, latex beads impregnated with a visual or a fluorescent dye or a combination thereof. Preferably is labelled with biotin. As used herein, the expression "labelled with biotin" means that the antibody is biotinylated.

In a preferred embodiment of the diagnostic and/or prognostic method of the first aspect, the sample is bronchoalveolar lavage fluid, knee synovial fluid, cell culture supernatant, peritoneal liquid, serum, plasma or urine. In a preferred embodiment, the sample is a tissue extract, a fluid such as bronchoalveolar fluid, knee synovial fluid, blood, serum, plasma, urine, peritoneal fluid, ascites, edema, cerebrospinal fluid, aqueous humour in the eye, serous fluid, perilymph or endolymph in the inner ear, saliva or cell culture supernatant, or extracellular vesicles obtained thereof. Preferably, the sample is bronchoalveolar lavage fluid, knee synovial fluid, cell culture supernatant, serum, plasma or urine.

In a preferred embodiment of the diagnostic and/or prognostic method of the first aspect, the sample is a human sample.

In a preferred embodiment of the diagnostic and/or prognostic method of the first aspect, the levels of CD5L are quantified. Even when what is detected are fragments of CD5L, what is quantified is the level of CD5L.

In a preferred embodiment of the diagnostic and/or prognostic method of the first aspect, the levels of CD5L are analysed by means of an Enzyme-Linked immunosorbent Assay (ELISA). For the analysis by ELISA, a standard curve for CD5L levels is used for comparing the CD5L level in the sample. ELISA is quantitative technique in which, in general, one of the antibodies is immobilized in a microplate (capture); the second antibody (detection) is linked generally to biotin, and is detected with peroxidase-linked streptavidin, which generates a colour change in the presence of its substrate. Following colour development, the concentration of the antigen is directly proportional to the colour intensity measured at 450 nm.

ELISA is a widely used technique in research to quantify specific proteins in fluids. Also, in the clinic, ELISA is used for the detection of circulating antibodies to disease-related antigens (e.g. infectious diseases such as Lyme disease -B. Burgdorferi-, HIV, Hepatitis, Herpes Simplex), or cardiac markers of heart attack among a long list of applications.

In a preferred embodiment of the diagnostic and/or prognostic method of the first aspect, the levels of CD5L are analysed by immunochromatography. Immunochromatography (IC) is a qualitative technique, faster and simpler (no additional reagents or instruments are needed). It comes in a strip format, in which the sample flows by capillarity, through a solid matrix. The most well-known immunochromatography test (ICT) is the pregnancy test sold in pharmacies. ICTs are commonly used for rapid medical diagnosis for at home or at points of care. They are already in use at the Spanish National Health Institute to detect PSA levels as a tumour marker, troponin I, HIV; and in the Emergency as rapid diagnostic tests for the Influenza virus type A and B, Streptococcus pneumoniae, Streptococcus group A β-haemolytic and Syncytial Respiratory Virus.

The goal of these tests is in many cases to provide for a complementary biomarker to clinical suspicion of pathology. This is in the case of the RPR IC test for Syphilis that detects anti-cardiolipin, cholesterol and lecitine IgM and IgG levels produced by damaged tissue, not those raised against the infectious agent.

These tests may not be highly specific but require 100% sensitivity. In contrast with prior methods and kits comprising antibodies for the diagnosis and/or prognosis of conditions that arise in an inflammatory context, this invention reaches 100% sensitivity and 80% specificity, which is a significantly higher value than the performances of methods and kits of the prior art.

In a second aspect, the present invention relates to a kit comprising the monoclonal antibody produced by the hybridoma deposited at the BCCM and assigned Accession No. LMBP 12584CB and the monoclonal antibody produced by the hybridoma deposited at the BCCM and assigned Accession No. LMBP 12583CB.

In a preferred embodiment of the present invention, the monoclonal antibody produced by the hybridoma deposited at the BCCM and assigned Accession No. LMBP 12583CB is conjugated or labelled, preferably is labelled with biotin. In a preferred embodiment of the kit of the second aspect, said kit further comprises a reactant which binds specifically to the label in the monoclonal antibody produced by the hybridoma deposited at the BCCM and assigned Accession No. LMBP 12583CB, preferably comprises streptavidin. In a preferred embodiment, the streptavidin is linked to an enzyme capable of turning a substrate into a coloured product. The enzyme is preferably a peroxidase, such as horseradish peroxidase, an alkaline phosphatase, etc. In a preferred embodiment, the enzyme is horseradish peroxidase.

In another preferred embodiment, said kit further comprises a solid matrix in which the sample can flow by capillarity.

**Table 2. Summary of the kits characteristics.**

| CHARACTERISTICS | ELISA KIT | ICT |
|---|---|---|
| Description: | Anti-CD5L in vitro diagnostic device using the ELISA to be used in human samples. | Anti-CD5L in vitro diagnostic device using IC to be used in human samples. |
| Method | ELISA | IC |
| Principle: | ELISA principle - Peroxidase conjugated | IC principle: latex or colloidal metal conjugated |
| Type of analysis: | quantitative | qualitative |
| Sample type: | any sample (plasma, serum, urine, saliva, etc.) | preferably plasma, serum, urine, saliva, peritoneal liquid |
| Biosafety | no additional biosafety in addition to Personal Protective Equipment | no additional biosafety in addition to Personal Protective Equipment |
| Time to result | 4 hours | 15-30 minutes |
| Reagent storage: | 4 ºC | room temperature |
| Training needs | Less than half a day for any level health care worker. | Less than 1 hour for any level health care worker |
| Equipment needed: | Spectrophotomer | no equipment needed |

In another preferred embodiment of the kit of the second aspect, the monoclonal antibody produced by the hybridoma deposited at the BCCM and assigned Accession No. LMBP 12584CB or the monoclonal antibody produced by the hybridoma deposited at the BCCM and assigned Accession No. LMBP 12583CB is conjugated to colloidal gold, colloidal silver, carbon, a visible or fluorescent dye, a magnetic particle, an enzyme, latex beads impregnated with a visual or a fluorescent dye or a combination thereof, preferably the monoclonal antibody produced by the hybridoma deposited at the BCCM and assigned Accession No. LMBP 12584CB or the monoclonal antibody produced by the hybridoma deposited at the BCCM and assigned Accession No. LMBP 12583CB is conjugated to colloidal gold or to latex beads impregnated with a visual dye.

A third aspect of the present invention relates to the use of the method of the first aspect, any one of the antibodies of the invention or the kit of the second aspect, for the diagnosis and/or prognosis of conditions that arise in an inflammatory context. Preferably, said use is for the diagnosis and/or prognosis of pathologies asthma, liver cirrhosis in Herpes C virus infection, liver cirrhosis and hepatocellular carcinoma in fatty liver disease, hepatocellular carcinoma in Herpes C virus infection, chronic liver disease due to Herpes C virus infection, hepatocellular carcinoma, cirrhosis, atopic dermatitis, hepatocellular carcinoma development in non-alcoholic steatohepatitis patients, Kawasaki disease, Turner syndrome, critical limb ischemia in diabetic patients, pulmonary tuberculosis, osteoarthritis, extreme physical stress, age-related macular degeneration, Crohn's disease, psoriatic arthritis, early onset preeclampsia, amyotrophic lateral sclerosis with cognitive impairment, prostate cancer, rapid decline in renal function in type 2 diabetes or, systemic lupus erythematosus.

In another preferred embodiment, said use is for the diagnosis and/or prognosis of asthma, liver damage, liver cirrhosis/fibrosis in Herpes C virus infection, liver cirrhosis/fibrosis and hepatocellular carcinoma in fatty liver disease, hepatocellular carcinoma in Herpes C virus infection, chronic liver disease due to Herpes C virus infection, hepatocellular carcinoma, response to sorafenib treatment in hepatocellular carcinoma, cirrhosis, atopic dermatitis, hepatocellular carcinoma development in non-alcoholic steatohepatitis patients, Kawasaki disease, Turner syndrome, critical limb ischemia in diabetic patients, tuberculosis, osteoarthritis, extreme physical stress, age-related macular degeneration, macular degeneration, Crohn's disease, psoriatic arthritis, early onset preeclampsia, amyotrophic lateral sclerosis with cognitive impairment, prostate cancer, renal function, kidney disease, graft-versus-host disease, rapid decline in renal function in type 2 diabetes. Preferably, for the diagnosis and/or prognosis of asthma, liver cirrhosis in Herpes C virus infection, liver cirrhosis and hepatocellular carcinoma in fatty liver disease, hepatocellular carcinoma in Herpes C virus infection, chronic liver disease due to Herpes C virus infection, hepatocellular carcinoma, cirrhosis, atopic dermatitis, hepatocellular carcinoma development in non-alcoholic steatohepatitis patients, Kawasaki disease, Turner syndrome, critical limb ischemia in diabetic patients, pulmonary tuberculosis, osteoarthritis, extreme physical stress, age-related macular degeneration, Crohn's disease, psoriatic arthritis, early onset preeclampsia, amyotrophic lateral sclerosis with cognitive impairment, prostate cancer, rapid decline in renal function in type 2 diabetes or systemic lupus erythematosus.

In another preferred embodiment, said use is for the diagnosis and/or prognosis of liver fibrosis/cirrhosis, hepatocellular carcinoma or tuberculosis.

In addition to being expressed in tissue under inflammatory conditions, CD5L is detected in high amounts in serum/plasma, where it circulates in association with IgM (Sanjurjo L et al. 2015). Interestingly, in chronic liver disease, high serum/plasma levels of this protein correlate with liver damage in Hepatitis C Virus (HCV) and Non Alcoholic Fatty Liver Disease (NAFLD) (Gangadharan B et al. 2007; Sarvari J et al. 2013; Mera, et al. 2014; Gray J et al. 2009) and in HCC (Yamazaki T et al. 2014).

The inventors have observed that CD5L plasma levels were significantly elevated in a cohort of HCV and alcoholic cirrhotic patients and that they correlated with advanced fibrosis.

Another aspect of the present invention is an antibody produced by the cell line deposited at the BCCM and assigned Accession No. LMBP 12584CB. Another aspect of the present invention is an antibody produced by the cell line deposited at the BCCM and assigned Accession No. LMBP 12583CB. Another aspect of the present invention relates to a kit comprising the monoclonal antibody produced by the hybridoma deposited at the BCCM and assigned Accession No. LMBP 12584CB. Another aspect of the present invention relates to a kit comprising the monoclonal antibody produced by the hybridoma deposited at the BCCM and assigned Accession No. LMBP 12583CB.

### DESCRIPTION OF THE DRAWINGS

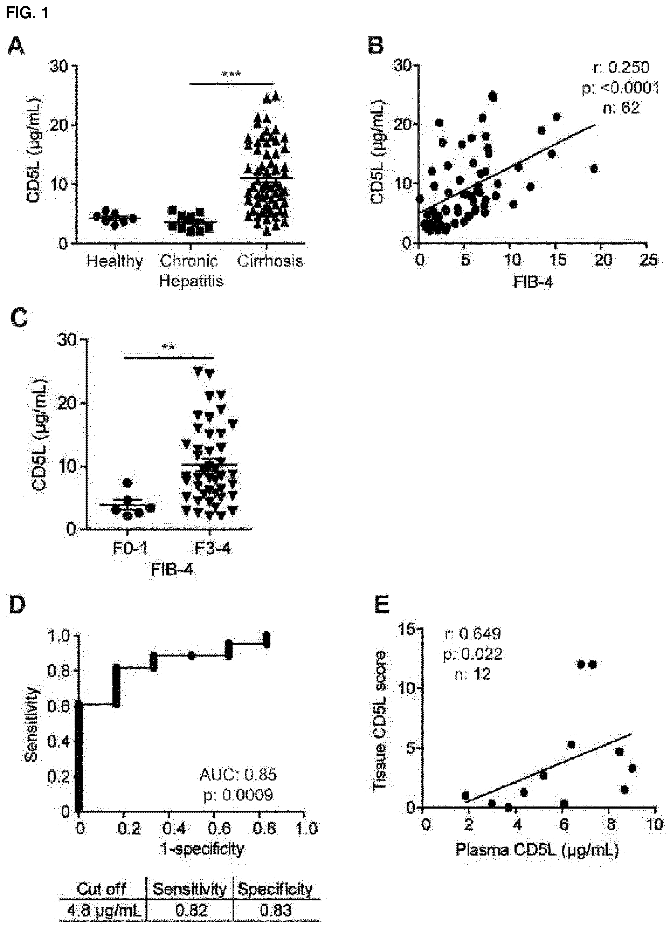

**Figure 1****.** Chronic liver damage induces hepatic CD5L expression. A) Plasma CD5L levels in function of disease stage (A) or FIB4 score (B). The horizontal lines indicate the mean values ± SEM for CD5L in each group. P values were calculated using Student's t test (***p≤0.001). B) Correlation of CD5L plasma levels with FIB-4 score of chronic hepatitis and cirrhotic patients. C) Plasma CD5L levels according to FIB-4 score categorized in F0-1 or F3-4. The horizontal lines indicate the mean values ± SEM for CD5L in each group. P value were calculated using the Mann-Whitney test (**p≤0.01). D) Receiver-operating characteristic (ROC) curve using CD5L levels for the discrimination of F0-1 vs. F3-4 patients.

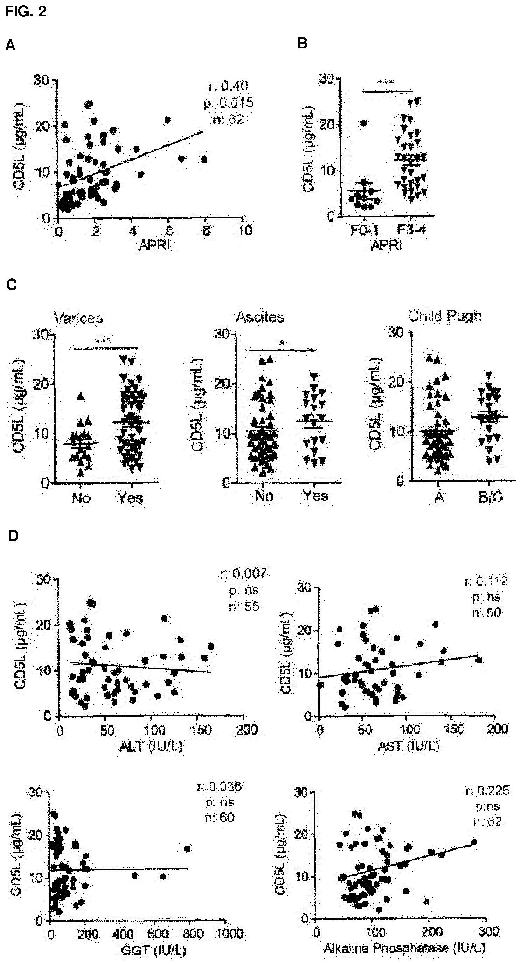

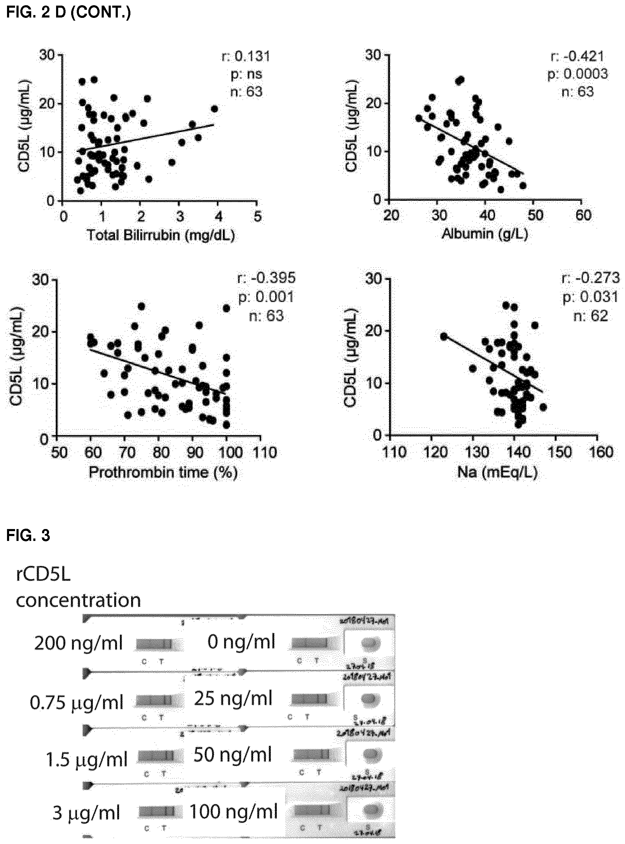

**Figure 2****.** Correlation of plasma CD5L concentrations and clinical parameters of cirrhosis severity. A) Correlation of CD5L plasma levels with APRI score of chronic hepatitis and cirrhotic patients. B) Plasma CD5L levels according to APRI score. The horizontal lines indicate the mean values ± SEM for CD5L in each group. P values were calculated using the Mann-Whitney test (*p≤0.05, **p≤0.01; ***p≤0.001). C) Plasma CD5L levels according to the presence of varicose veins, ascites, or Child Pugh B/C vs. A. The horizontal lines indicate the mean values ± SEM for CD5L in each group. P values were calculated using the Mann-Whitney test (*p≤0.05, **p≤0.01). D) Correlation of CD5L concentrations with clinical parameters, as indicated.

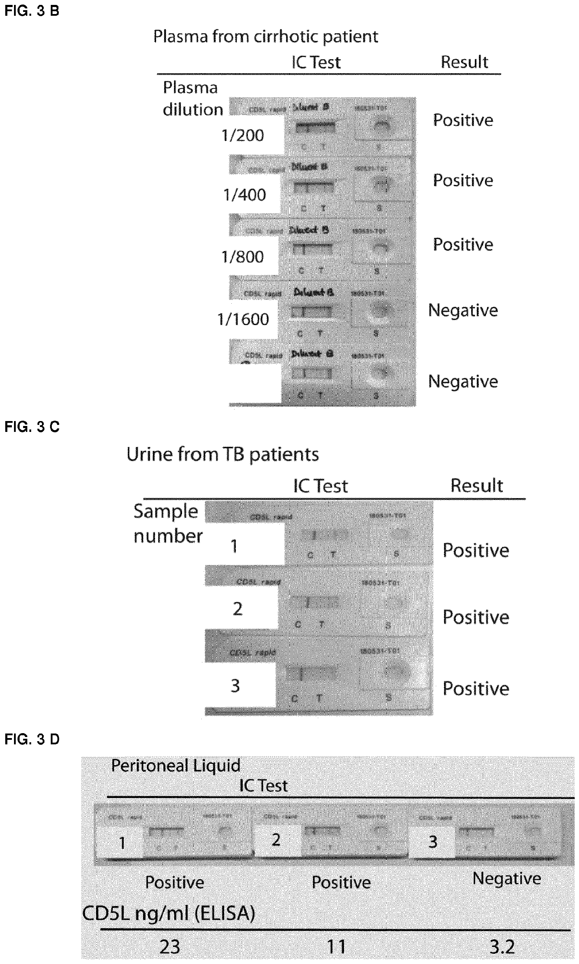

**Figure 3****.** Levels of CD5L protein as determined using an IC test manufactured with antibodies deposited at the BCCM and assigned Accession No. LMBP 12584CB, and LMBP 12583CB. A) Indicated concentrations or recombinant CD5L (rCD5L) protein. B) Indicated dilutions of plasma from a cirrhotic patient. C) Urine from 3 different TB patients. D) IC test of peritoneal liquid from 3 different donors (upper panel), and CD5L concentrations as determined by ELISA (lower data).

### EXAMPLES

The following examples illustrate the present invention and demonstrate the advantageous properties of the method, monoclonal antibodies and kits of the present invention.

### Example 1: Production of recombinant human CD5L

Recombinant CD5L was produced as previously described (Sanjurjo L. et al. 2015): The cDNA of human CD5L (CD5L) was obtained by gene synthesis (GenScript) following NCBI reference sequence NP_005885.1, with a modification in which the Immunoglobulin G chain signal peptide replaced that of CD5L. The sequence of this recombinant CD5L is SEQ ID NO: 1. The cDNA was cloned into the p.evi vector and transiently transfected into CHO K1 cells using the eviFect system (Evitria AG). Cells were grown in eviMake, a chemically defined, serum-free, animal component-free medium. The cell culture supernatant was harvested at day 8 after transfection, dialyzed to 20 mM Na₂HPO₄, pH 7.4 and subjected to MonoQ chromatography. Recombinant CD5L was eluted in a sodium chloride gradient, and purification was monitored by SDS-PAGE.

Purified protein was dialyzed to PBS, concentrated by centrifugation on Amicon ultra (Millipore, UFC901024), and possible endotoxin contamination was removed by Endotrap columns (Hyglos GmbH, 321063), following the manufacturer's protocol.

As mentioned above, the purified rCD5L was tested in preliminary experiments, where its activity in terms of MΦ TNFα secretion, as well as inhibition of apoptosis induced by cycloheximide treatment, was comparable to that of commercially available rCD5L (R&D Systems).

### Example 2: Monoclonal antibodies (mAbs) generation and enzyme-linked immunosorbent assay (ELISA) assays.

Murine mAbs against human CD5L were raised by subcutaneous immunization of BALB/c mice with 25 µg of a KLH-coupled rCD5L, in 0.15 mL of sterile PBS emulsified with the same volume of Freund's complete adjuvant (Difco). Mice were boosted subcutaneously on days 28 and 56 with the same amount of protein in Freund's incomplete adjuvant. Serum from immunized mice was collected 10 days after the last boost, and the presence of specific antibodies against rCD5L was determined by ELISA.

Selected mice were boosted intravenously with 30 µg of KLH-conjugated protein in sterile PBS three days before fusion of spleen lymphocytes with the P3X63Ag8.653 murine plasmacytoma (CRL1580, American Type Culture Collection) using polyethylene glycol 4000 (Merck). Cell fusion was performed using standard procedures (Galfre G et al. 1977; Harlow E L DE. 1988).

Two weeks after fusion, culture supernatants were screened in ELISA for the presence of CD5L-specific antibodies, using bovine serum albumin as negative control. Positive hybridomas were cloned by limiting dilution. mAbs were produced in tissue culture supernatants and purified by affinity chromatography using Protein G-Sepharose (GE Healthcare). The immunoglobulin subclass was determined by ELISA using specific peroxidase-conjugated antibodies against the heavy chain of mouse immunoglobulins (IgG1, IgG2a, IgG2b, IgG3 and IgM). The two mAbs were IgG1.

EZ-Link PEO-maleimide-activated biotin (Pierce, Perbio Science,Cheshire, UK) was dissolved in PBS at a concentration of 10 mM and then added to mAb solutions at a molar ratio of 5:1. The reaction mixtures were incubated for 30 min at room temperature and then desalted and exchanged with PBS over a Hi TrapTM desalting column (Amersham Pharmacia Biotech) to remove free biotin. To screen anti-CD5L mAb, 100 ml of serum-free culture supernatant from cell transfectants expressing rCD5L or BSA was used to coat 96-well microtiter plates (Nunc, Denmark) overnight at 4 ºC. Plates were blocked with PBS containing 5 % BSA for 1 h at 22 ºC. Hybridoma supernatant was then added to the wells and incubated for 1 h at 22 ºC. MAbs that reacted against rCD5L supernatant, but not against BSA, were considered positive.

For sandwich ELISA assays, 500 ng of mAb LMBP 12584CB was coated onto a microtiter plate overnight at 4 ºC. The plates were blocked for 1 h at 22 ºC with PBS containing 5 % BSA. Various concentrations of plasma or affinity-purified rCD5L (used as standard for quantitation) were then added and incubated for 1.5 h at 22 ºC. Finally, 100 ng of biotinylated mAb LMBP 12583CB was added for 1 h at 22 ºC.

For all the ELISA assays, the plates were washed five times with PBS 0.01 % Tween-20 to remove unbound proteins. For bound protein detection, a 1:1000 dilution of peroxidase (PO)-labeled anti-mouse IgG antibody (for unlabeled mAb detection) or PO-labeled streptavidin (for biotinylated mAb detection) was added in the final step and incubated for 30 min at 22 ºC (both from Sigma). Unbound PO antibody/streptavidin was washed five times with PBS 0.01 % Tween-20. Color was developed by adding 3,3',5,5'-tetramethylbenzidine liquid substrate (Sigma), and the optical density was read at 450 nm using a Varioskan Flash microplate reader (ThermoFisher Scientific Inc). Each assay was repeated three times, with similar results. For sandwich ELISA of human plasma, results were validated by western blotting in a subset of samples. The lower limit detection of the ELISA was 0.625 ng/mL. The measurement range was 0.625 to 80 ng/mL, with an intra- and interassay precision of 2.8 % and 2.2 %, respectively.

### Example 3: CD5L is a marker of liver fibrosis

Plasma CD5L levels were quantified in patients with different stages of chronic liver disease, ranging from chronic hepatitis to cirrhosis. The baseline clinical characteristics of these patients are shown in table 3. Plasma CD5L concentration in patients with cirrhosis was approximately 3-fold higher than in those with chronic hepatitis and in healthy individuals (mean µg/ml ± SD, Cirrhotic, 11 ± 5.7; chronic hepatitis 3.6 ± 1.2; healthy 4.2 ±0.8) (Figure 1A). Furthermore, CD5L levels positively correlated with increased fibrosis scores, namely FIB4 (Figure 1B) and APRI (Figure 2A). Moreover, CD5L levels in patients with advanced fibrosis (F3-4) were significantly higher than those in non-fibrosis or mild fibrosis (F0-1) patients, as determined by FIB4 (Figure 1C) and APRI scores (Figure 2B). We analyzed the sensitivity and specificity of CD5L to discriminate advanced (F3-4) vs. mild fibrosis (F0-1) using the ROC curve and the AUROC (Figure 1D). The best cut-off value corresponded to a concentration of 4.8 µg/ml, with a sensitivity of 0.82 and a specificity of 0.83. CD5L plasma levels also associated with complications of cirrhosis, since they were higher in those patients with esophagic or gastric varices and with ascites compared with those without (p=0.0017 and p=0.0327, respectively) (Figure 2C). Moreover, in cirrhotic patients, we observed a negative correlation of CD5L plasma levels with albumin, prothrombin time, sodium levels, and platelet counts (Figure 2D). These results suggest that plasma CD5L is strongly associated with disease severity.

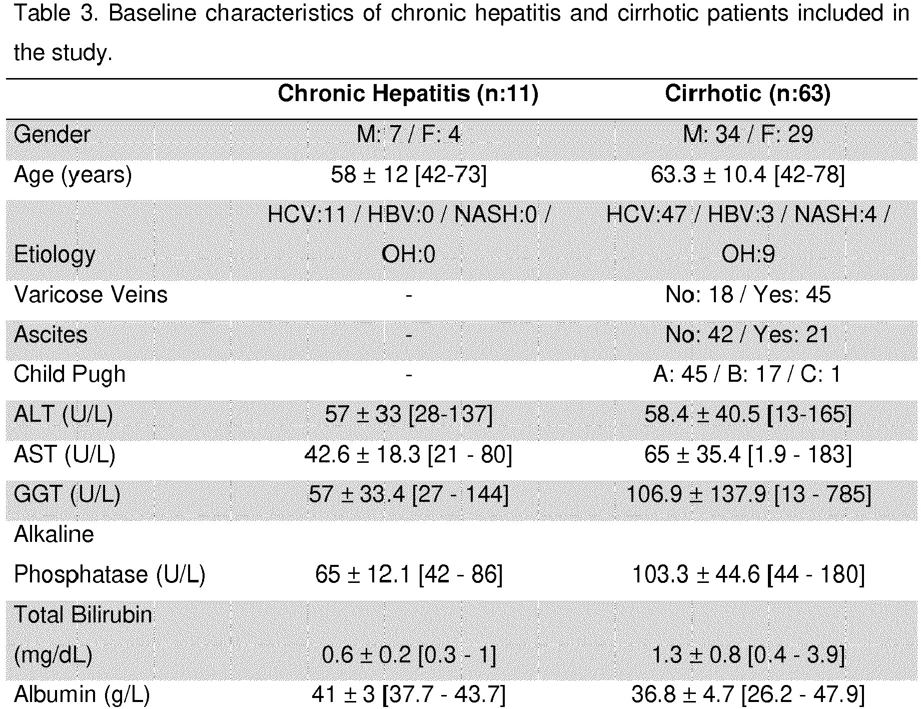

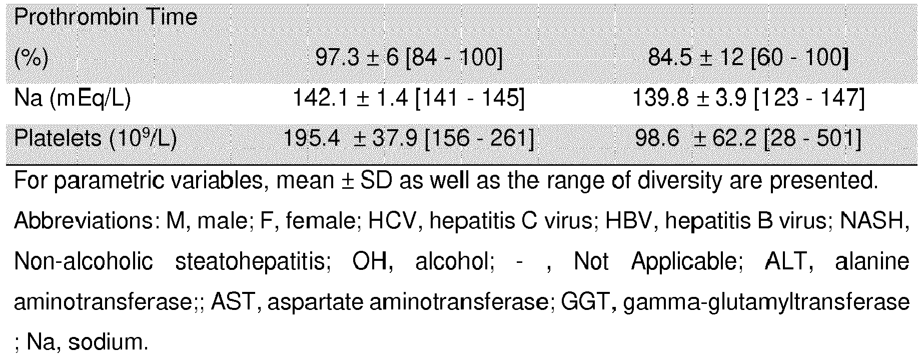

### Example 4: human CD5L ELISA kit protocol and performance analysis

Reagents:
- Coating Buffer: 0.1 M Sodium Carbonate Na₂CO₃ pH 9.5
- Blocking buffer (BB): PBS (10 mM Na₂HPO₄, 150 Mm NaCl, pH 7.4) with 5 % Bovine serum albumin (BSA)
- Wash buffer: PBS with 0.05 % tween-20
- Capture Antibody: Human anti-CD5L (clone LMBP 12584CB)
- Standard: rCD5L
- Detection Antibody: Biotin-conjugated anti-CD5L (Clone LMBP 12583CB)
- Streptavidin HRP conjugated (SA-HRP)
- Substrate solutions (TMB): 3,3',5,5'-Tetramethylbenzidine
- Stop solution: 2 M H₂SO₄

### Preparation of working solutions

Standard preparation: 500 µL of BB were added to 4 µL of rCD5L and mixed gently. The concentration of the rCD5L was 1.6 µg/ml and it is hereafter referred to as a Master Standard. From this Master Standard, a series of dilutions were produced as explained in table 4. A standard of 80 ng/mL was prepared and serial dilutions were performed in the plate.

**Table 4. Standard preparation.**

| Standard | Volume | BB | CD5L ng/ml |
|---|---|---|---|
| 1 | 40 µL of Master Standard | 360 µL | 80 |
| 2 | 50 µL of Standard 1 | 50 µL | 40 |
| 3 | 50 µL of Standard 2 | 50 µL | 20 |
| 4 | 50 µL of Standard 3 | 50 µL | 10 |
| 5 | 50 µL of Standard 4 | 50 µL | 5 |
| 6 | 50 µL of Standard 5 | 50 µL | 2,5 |
| 7 | 50 µL of Standard 6 | 50 µL | 1,25 |
| 8 | 50 µL of Standard 7 | 50 µL | 0,625 |
| Blank | | 50 µL | 0 |

### Sample preparation:

Human samples were diluted in BB as follows: plasma samples were diluted 400-fold, and urine, cell culture supernatants and peritoneal liquid, 2-fold. Samples from synovial fluid, ascites, saliva, eye drops are diluted similarly.

Detection antibody LMBP 12583CB was prepared to have a concentration of 1 µg/mL in BB at the time of assay.

HRP conjugated streptavidin 1:1000 in BB was prepared at time of assay and kept in the dark.

### Procedure:

1. Wells were coated with 50 µL per well of Capture Antibody (LMBP 12584CB) at 5 µg/mL diluted in coating buffer. 50 µL of diluted antibody were added per well, the plate was sealed and incubated overnight at 4ºC.
2. The plate was blocked with 200 µL of blocking buffer at RT for 1 hour.
3. Standard and samples were added to the plate (50 µL /well) at room temperature for 1.5 hours.
4. The wells were washed five times with 200 µL /well with wash buffer.
5. 1 µg/mL of detection antibody was added in 50µL/well in blocking buffer and incubated at room temperature for 1 hour.
6. The wells were washed five times with 200 µL /well with wash buffer.
7. 50 µL of SA-HRP, diluted 1/1000 in blocking buffer, were added to each well and incubated at room temperature and in the dark for 30 minutes.
8. The wells were washed five times with 200 µL /well with wash buffer.
9. 50 µL of substrate solution (TMB) were added to each well and the plate was incubated at room temperature in the dark for 8-12 minutes.
10. The absorbance at 560 nm was quantified.
11. 50 µL of Stop Solution were added to each well and absorbance was quantified at 450 nm.

### Assay characteristics

1. Sensitivity: The lower limit detection is 0.625 ng/mL of CD5L. The measure range is 0.625 to 80 ng/mL. Any samples reading higher than the highest standard should be diluted with dilution buffer in higher dilution and re-assayed.
2. Specificity: human
3. Intra-assay precision: three samples were tested three times on one plate to assess intra-assay precision.

**Table 5. CD5L concentrations in ng/mL.**

| **Sample** | **Result 1** | **Result 2** | **Result 3** | **Mean** | **SD** | **% CV** | **Inter assay CV (%)** |
|---|---|---|---|---|---|---|---|
| **Test 1** | | | | | | | |
| 1 | 4042.15 | 3717.69 | 4200.37 | 3986.74 | 246.06 | 3.09 | |
| 2 | 17188.62 | 17972.59 | 16207.16 | 17122.79 | 884.55 | 2.58 | |
| 3 | 5628.70 | 5539.65 | 6029.26 | 5732.54 | 260.80 | 2.28 | |

| **Test 2** | | | | | | | |
|---|---|---|---|---|---|---|---|
| 1 | 5256.83 | 4316.94 | 5060.11 | 4877.96 | 495.71 | 5.08 | |
| 2 | 16469.95 | 16120.22 | 15857.92 | 16149.36 | 307.05 | 0.95 | |
| 3 | 7857.92 | 7530.06 | 7114.75 | 7500.91 | 372.44 | 2.48 | |

| **Test 3** | | | | | | | |
|---|---|---|---|---|---|---|---|
| 1 | 4424.91 | 3830.23 | 3926.89 | 4060.68 | 319.12 | 3.93 | |
| 2 | 16403.87 | 16160.29 | 16351.19 | 16305.12 | 128.16 | 0.39 | |
| 3 | 8584.55 | 7856.94 | 7101.97 | 7847.82 | 741.34 | 4.72 | **2.83** |

4. Inter-assay precision: three samples were tested in three separate assays to assess inter-assay precision.

**Table 6. CD5L concentrations in ng/mL.**

| | **Result 1** | **Result 2** | **Result 3** | **Mean** | **SD** | **%CV** | **Interassay CV (%)** | |
|---|---|---|---|---|---|---|---|---|
| **Sample 1** | | | | | | | | |
| ELISA 1 | 4042.15 | 3717.69 | 4200.37 | 3986.74 | 200.91 | 2.52 | | |
| ELISA 2 | 5256.83 | 4316.94 | 5060.11 | 4877.96 | 404.75 | 4.15 | | |
| ELISA 3 | 4424.91 | 3830.23 | 3926.89 | 4060.68 | 260.56 | 3.21 | **3.29** | |

| **Sample 2** | | | | | | | | |
|---|---|---|---|---|---|---|---|---|
| ELISA 1 | 17188.62 | 17972.59 | 16207.16 | 17122.79 | 722.24 | 2.11 | | |
| ELISA 2 | 16469.95 | 16120.22 | 15857.92 | 16149.36 | 250.71 | 0.78 | | |
| ELISA 3 | 16403.87 | 16160.29 | 16351.19 | 16305.12 | 104.64 | 0.32 | **1.07** | |

| **Sample 3** | | | | | | | | |
|---|---|---|---|---|---|---|---|---|
| ELISA 1 | 5628.70 | 5539.65 | 6029.26 | 5732.54 | 212.94 | 1.86 | | |
| ELISA 2 | 7857.92 | 7530.06 | 7114.75 | 7500.91 | 304.10 | 2.03 | | TOTAL |
| ELISA 3 | 8584.55 | 7856.94 | 7101.97 | 7847.82 | 605.30 | 3.86 | **2.31** | **2.23** |

5. Linearity: to assess the linearity of the assay, three samples were serially diluted (1/2) with the Blocking Buffer to produce samples with values within the dynamic range of the assay. The initial plasma dilutions were 1/400.

One of the advantages of the present invention is that it is characterized by working for several kinds of samples, including plasma samples. The inventors have successfully tested the kit of the invention in several samples, and some of them, containing CD5L are:
- Cell culture supernatants;
- Plasma;
- Serum;
- Urine
- Peritoneal liquid

Moreover, the inventors believe that other kind of samples would be suitable to be tested by the kit of the invention, for example:
- Synovial fluid;
- Ascites;
- Saliva;
- Eye drops; and
- Other fluids

### Example 5: Immunochromatography tests analysing CD5L levels in human fluids

Plasma, urine or peritoneal liquid were dispensed into sample well and allowed to diffuse through the membrane in which the monoclonal antibody produced by the hybridoma deposited at the BCCM and assigned Accession No. LMBP 12584CB had been immobilized (test line, T). During the assay, the diluted sample reacted with color-conjugated monoclonal antibody produced by the hybridoma deposited at the BCCM and assigned Accession No. LMBP 12583CB. If the concentration of CD5L in the sample was above 5 ng/ml, a colored line with a specific antibody-CD5L-conjugate formed within 20 mins at the test line region (T). No colored line was visible in the T line in samples in which concentrations were below 5 ng/ml, suggesting a negative result. In all cases, red color appeared at the control line (C), indicating that the test was performed correctly (figure 3).

### Bibliography:

Sanjurjo L et al. J Leukoc Biol. 2015;98:173-84.
Tissot JD et al. J Immunol Methods. 1994;173:63-75.
Yamazaki T et al. PLoS One. 2014;9:e109123.
Sanjurjo L, et al. PLoS One. 2013;8:e79670.
Kim WK, et al. Exp Mol Med. 2008;40:677-85.
Yu HR, et al. Pediatr Allergy Immunol. 2009;20:699-707.
Balakrishnan L, et al. Clin Proteomics. 2014;11:1.
Armengol C, et al. Crit Rev Immunol. 2013;33:57-96.
Wu, J. et al. Mol Cell Proteomics. 2005;4:1251-64.
Gangadharan B. et al. Clin Chem. 2007;53:1792-9.
Gray, J. et al. BMC Cancer. 2009;9:271.
Sarvari, J. et al. Hepat Mon. 2013;13:e8351.
Mera K. et al.. BMC Gastroenterol. 2014;14:1-10.
Koyama et al. Journal of Gastroenterol. 2018; 53: 770-779.
Kolialexi A. et al. J Proteome Res. 2010;9:5164-70.
Hung, P-H. et al. Mol Biosyst. 2011;7:1990-8.
Xu, DD, et al. Proteomics. 2014;14:322-31.
Balfoussia E. et al. J Proteomics. 2014;98:1-14.
Iannacone A, et al. Experimental Eye Research. 2017; 155;64-74.
Ono Y, et al. BMC Gastroenterology; 2017;17:1-9
Cretu D et al. Arthritis Care Res . 2017;70:454-61.
Kolialexi A, et al. Expert Rev Proteomics; 2017;14:269-76.
Xu Z et al. Amyotroph Lateral Scler Front Degener; 2018;0:1-15
Xiaofei Lai et al, Int Immunopharmacol 2018;63:311-6.
Galfre G, et al. Nature. 1977;266:550-2.
Harlow E L DE. Antibodies: A Laboratory Manual. Cold Spring Harbor, New York: Cold Spring Harbor Laboratory Press.; 1988.

## Claims

1. A diagnostic and/or prognostic method comprising:
a. analysing the levels of CD5L in a sample using the monoclonal antibody produced by the hybridoma deposited at the BCCM and assigned Accession No. LMBP 12584CB and the monoclonal antibody produced by the hybridoma deposited at the BCCM and assigned Accession No. LMBP 12583 CB;
b. comparing the levels of CD5L obtained in step (a) with those analysed in control samples; and
c. arriving to a diagnosis/prognosis where the levels in the sample are consistent with those in subjects who have been diagnosed previously.

2. The method according to the preceding claim, wherein the monoclonal antibody produced by the hybridoma deposited at the BCCM and assigned Accession No. LMBP 12583CB is labelled with biotin, conjugated to colloidal gold, colloidal silver, carbon, a visible or fluorescent dye, a magnetic particle, an enzyme such as Horseradish peroxidase, alkaline phosphatase, latex beads impregnated with a visual or a fluorescent dye or a combination thereof; preferably is labelled with biotin.

3. The method according to any one of the preceding claims, wherein the sample is bronchoalveolar lavage fluid, knee synovial fluid, cell culture supernatant, peritoneal liquid, serum, plasma or urine.

4. The method according to any one of the preceding claims, wherein the levels of CD5L are quantified.

5. The method according to any one of the preceding claims, wherein the levels of CD5L are analysed by means of an Enzyme-Linked immunosorbent Assay (ELISA).

6. The method according to any one of claims 1 to 3, wherein the levels of CD5L are analysed by immunochromatography.

7. An antibody produced by the cell line deposited at the BCCM and assigned Accession No. LMBP 12584 CB.

8. A kit comprising the monoclonal antibody produced by the hybridoma deposited at the BCCM and assigned Accession No. LMBP 12584CB and the monoclonal antibody produced by the hybridoma deposited at the BCCM and assigned Accession No. LMBP 12583 CB.

9. The kit according to the preceding claim, wherein the monoclonal antibody produced by the hybridoma deposited at the BCCM and assigned Accession No. LMBP 12583CB is conjugated or labelled, preferably is labelled with biotin.

10. The kit according to the preceding claim, wherein said kit further comprises a reactant which binds specifically to the label in the monoclonal antibody produced by the hybridoma deposited at the BCCM and assigned Accession No. LMBP 12583CB, preferably comprises streptavidin.

11. The kit according to claim 8, wherein said kit further comprises a solid matrix in which the sample can flow by capillarity.

12. The kit according to any one of claims 8 or 11, wherein the monoclonal antibody produced by the hybridoma deposited at the BCCM and assigned Accession No. LMBP 12584CBor the monoclonal antibody produced by the hybridoma deposited at the BCCM and assigned Accession No. LMBP 12583CB is conjugated to colloidal gold, colloidal silver, carbon, a visible or fluorescent dye, a magnetic particle, an enzyme, latex beads impregnated with a visual or a fluorescent dye or a combination thereof, preferably the monoclonal antibody produced by the hybridoma deposited at the BCCM and assigned Accession No. LMBP 12584CB or the monoclonal antibody produced by the hybridoma deposited at the BCCM and assigned Accession No. LMBP 12583CB is conjugated to colloidal gold or to latex beads impregnated with a visual dye.

13. Use of the method according to any one of claims 1 to 6, the antibody according to claim 8 or the kit according to any one of claims 8 to 12, for the diagnosis and/or prognosis of conditions that arise in an inflammatory context.

14. The use according to the preceding claim for the diagnosis and/or prognosis of pathologies asthma, liver cirrhosis in Herpes C virus infection, liver cirrhosis and hepatocellular carcinoma in fatty liver disease, hepatocellular carcinoma in Herpes C virus infection, chronic liver disease due to Herpes C virus infection, hepatocellular carcinoma, cirrhosis, atopic dermatitis, hepatocellular carcinoma development in non-alcoholic steatohepatitis patients, Kawasaki disease, Turner syndrome, critical limb ischemia in diabetic patients, pulmonary tuberculosis, osteoarthritis, extreme physical stress, age-related macular degeneration, Crohn's disease, psoriatic arthritis, early onset preeclampsia, amyotrophic lateral sclerosis with cognitive impairment, prostate cancer, rapid decline in renal function in type 2 diabetes or systemic lupus erythematosus, preferably for the diagnosis and/or prognosis of liver fibrosis/cirrhosis, hepatocellular carcinoma or tuberculosis.

15. The use according to any one of the two preceding claims for the diagnosis and/or prognosis of liver fibrosis/cirrhosis, hepatocellular carcinoma or tuberculosis.
